# EUROPÄISCHE PATENTSCHRIFT

(11) **EP 2 900 184 B1**
(45) Veröffentlichungstag und Bekanntmachung des Hinweises auf die Patenterteilung: **14.09.2016**
(21) Anmeldenummer: 13765950.4
(22) Anmeldetag: 18.09.2013
(51) Int. Cl.: A61F 9/007

(54) **HANDSTÜCK FÜR DIE PHAKOEMULSIFIKATION EINER AUGENLINSE**
HANDPIECE FOR THE PHACOEMULSIFICATION OF AN EYE LENS
PIÈCE À MAIN POUR LA PHACOÉMULSIFICATION D'UN CRISTALLIN OCULAIRE

(30) Priorität: 28.09.2012 DE 102012019165
(43) Veröffentlichungstag der Anmeldung: 05.08.2015
(73) Patentinhaber: Carl Zeiss Meditec AG, 07745 Jena (DE)
(72) Erfinder: KUEBLER, Christoph, 73447 Oberkochen (DE); WEHNER, Wolfram, 90402 Nürnberg (DE); REIN, Karlheinz, 73430 Aalen (DE)
(74) Vertreter: Carl Zeiss AG - Patentabteilung
(86) Internationale Anmeldenummer: PCT/EP2013/002805
(87) Internationale Veröffentlichungsnummer: WO 2014/048550

(56) Entgegenhaltungen:
- WO-A1-2010/094353
- WO-A2-2012/041288
- US-A1- 2011 218 483
- US-A1- 2011 301 469

## Beschreibung

Die Erfindung betrifft ein Handstück für die Phakoemulsifikation einer Augenlinse.

Zur Behandlung einer Augenlinsentrübung, welche in der Medizin als grauer Star bezeichnet wird, gibt es mehrere chirurgische Techniken. Die am weitesten verbreitete Technik ist die Phakoemulsifikation, bei der eine dünne Nadel in die Augenlinse eingeführt und mit Ultraschall zu Schwingungen angeregt wird. Die vibrierende Nadel emulsifiziert in ihrer nächsten Umgebung die Linse derart, dass die entstehenden Linsenpartikel durch eine Aspirationsleitung mittels einer Pumpe abgesaugt werden können. Dabei wird ein Spülfluid (Irrigationsfluid) zugeführt, wobei das Absaugen der Partikel und des Fluides durch die Aspirationsleitung erfolgt, welche üblicherweise innerhalb der Nadel angeordnet ist. Ist die Linse vollständig emulsifiziert und entfernt worden, kann in den leeren Kapselsack eine neue künstliche Linse eingesetzt werden, sodass ein derart behandelter Patient wieder ein gutes Sehvermögen erreichen kann.

Während der Emulsifikation der Augenlinse mittels Ultraschall reibt die schwingende Nadel und eine die Nadel umgebende Hülse am Gewebe des zu behandelnden Auges, so dass sich der Kontaktbereich stark erwärmen kann. Bei längerem Betrieb der schwingenden Nadel kann der Gewebebereich um die Nadel herum verbrennen. Da die Nadel durch die Hornhaut gestochen wird, bedeutet dies, dass vor allem die Hornhaut thermisch sehr stark belastet wird. Eine auch nur lokal verbrannte Hornhaut wird als schwere Komplikation eingestuft, welche unbedingt vermieden werden muss.

In DE 10 2010 047 010 A1 ist eine Steuerungsvorrichtung für ein ophthalmochirurgisches System beschrieben, welches eine Messvorrichtung zum Ermitteln des Volumens einer Augenvorderkammer eines während einer Phakoemulsifikation zu behandelnden Auges und eine Steuereinheit aufweist, welche in Abhängigkeit von dem ermittelten Volumen der Augenvorderkammer eine Steuergröße berechnet, mit der sich ein Druck oder ein Volumenstrom in einer Irrigationsleitung und/oder Aspirationsleitung des ophthalmochirurgischen Systems steuern lässt.

In US 2011/0218483 A1 ist ein Phakoemulsifikations-Handstück beschrieben, welches eine Nadel und ein daran gekoppeltes mikroelektromechanisches System (MEMS) aufweist. Das Handstück kann ferner ein Horn aufweisen, wobei das Horn mit der Nadel und dem MEMS gekoppelt ist. Das MEMS ist so eingerichtet, dass es eine Bewegung der Nadel in mindestens eine Richtung erzeugen kann.

US 2010/0094321 A1 beschreibt ein Ultraschall-Handstück für die Phakoemulsifikation, welches mindestens ein Paar schwingender Piezoelemente aufweist, welche innerhalb des Handstückes so angeordnet sind, dass sie Schwingungen erzeugen, welche senkrecht oder in einem Winkel geneigt zur Längsachse des Handstückes orientiert sind.

WO 2010/094353 lehrt ein Handstück gemäß dem Oberbegriff des Anspruchs 1.

Es ist eine Aufgabe der Erfindung, ein Handstück für die Phakoemulsifikation zu schaffen, mit dem eine Emulsifikation der Linse auch bei längerer Operationsdauer nur mit einer geringen oder gar keinen Temperaturbelastung des Auges erreicht werden kann.

Die Aufgabe wird durch den Gegenstand des unabhängigen Patentanspruchs 1 gelöst. Vorteilhafte Weiterbildungen der Erfindung sind Gegenstand der Unteransprüche.

Das erfindungsgemäße Handstück für die Phakoemulsifikation einer Augenlinse weist auf:
- eine Nadel mit einer darin angeordneten Aspirationsleitung zum Transportieren von Aspirationsfluid, und
- mindestens ein Antriebselement zum Erzeugen einer Longitudinalwelle,
wobei das Antriebselement so angeordnet ist, dass sich die Longitudinalwelle direkt in das Aspirationsfluid einkoppeln lässt.

Bei Handstücken gemäß dem Stand der Technik bewirkt ein Antriebselement, dass die von ihm erzeugte Longitudinalwelle vollständig zur Bewegung der Nadel verwendet wird. Die Longitudinalwelle wird mit hoher Frequenz, z.B. mit einer Resonanzfrequenz des Antriebselementes in Höhe von etwa 40.000 Hz, erzeugt, so dass die derart in Schwingungen versetzte Nadel zum Zertrümmern der Augenlinse im Bereich des distalen Endes der Nadel verwendet wird. Bei dem erfindungsgemäßen Handstück lässt sich hingegen die von einem Antriebselement erzeugte Longitudinalwelle direkt in das Aspirationsfluid einkoppeln. Die durch die Longitudinalwelle übertragene kinetische Energie des schwingenden Antriebselementes wird somit genutzt, um einen Fluiddruck zu erzeugen, welcher ein Zertrümmern der zu behandelnden Augenlinse bewirkt. Damit wird die von dem Antriebselement abgegebene Energie durch das Aspirationsfluid übertragen, so dass keine oder nur wenig Energie, z.B. 30 % der Energie, für eine Bewegung der Nadel zur Verfügung steht. Dies hat zur Folge, dass in der Umgebung der Nadel keine oder nur wenig kinetische Energie in Reibungswärme umgesetzt wird, so dass keine Temperaturbelastung des die Nadel umgebenden Gewebes auftreten kann.

Bevorzugt lässt sich die gesamte von dem Antriebselement abgegebene Energie in das Aspirationsfluid einkoppeln, so dass die Nadel während der Phakoemulsifikation vollständig unbewegt bleibt. Da es somit keinerlei Relativbewegung zwischen der Nadel und dem der Nadel umgebenden Gewebe mehr gibt, entsteht auch keinerlei Erwärmung in dem umgebenden Gewebe. Die von dem Antriebselement in Form von Longitudinalwellen abgegebene Energie wird dann ausschließlich über Aspirationsfluid bis zum distalen Ende der Nadel an die zu behandelnde Augenlinse transportiert. Damit wir ein sehr hoher Wirkungsgrad erreicht.

Allgemein tritt durch das erfindungsgemäße Handstück keine Traumatisierung oder Zerstörung sensiblen Gewebes in Bereichen des Auges mehr auf, welche nicht das distale Ende der Nadel sind. Dies ist besonders bei einer Verstopfung (Okklusion) der Aspirationsleitung vorteilhaft. In diesem Fall wird keine Energie mehr in das die Nadel umgebende Gewebe und auch nicht in die Umgebung des distalen Endes der Nadel abgestrahlt. Sämtliche Longitudinalwellen gelangen nur bis zu dem die Aspirationsleitung verstopfenden Linsenpartikel. Die Energie wirkt also nur dort, wo sie tatsächlich benötigt wird.

Gemäß einer bevorzugten Ausführungsform der Erfindung ist das Antriebselement innerhalb der Aspirationsleitung angeordnet und lässt sich in direkten Kontakt mit dem Aspirationsfluid bringen. Damit kann eine sehr einfache Konstruktion erreicht werden, bei der keine Dämpfungsverluste durch zwischengelagerte Elemente entstehen. Durch einen solchen Aufbau wird der Wirkungsgrad des Handstückes nochmals erhöht.

Vorzugsweise weist das Antriebselement piezoelektrische Elemente auf. Damit sind hohe Frequenzen bis zu 40000 Hz möglich, wobei durch eine geschickte Anordnung der piezoelektrischen Elemente relativ hohe Stellwege für die Longitudinalbewegung möglich sind.

Das Antriebselement ist bevorzugt ringförmig ausgebildet. Damit kann das abzusaugende Aspirationsfluid durch die durch den Ring gebildete zentrale Ausnehmung abgeführt werden. Das Aspirationsfluid muss somit vom distalen Ende der Nadel beginnend nicht über Umwege entlanggeführt werden, sondern kann zentral durch das ganze Handstück geleitet werden. Damit ist eine einfache und platzsparende Konstruktion möglich.

Bevorzugt weist die Nadel einen trichterförmigen Bereich mit einem sich verjüngenden Ende auf, welches zum distalen Ende der Nadel orientiert ist. Durch einen trichterförmigen Bereich können die Longitudinalwellen in Richtung zum sich verjüngenden Ende eine höhere Geschwindigkeit erreichen, so dass am distalen Ende der Nadel ein hoher Schalldruckpegel vorliegen kann.

Der trichterförmige Bereich kann im Querschnitt eine Innenwandkontur aufweisen, welche zur Mittelache der Aspirationsleitung hin so gekrümmt ist, dass ein zugehöriger Krümmungsmittelpunkt zum verjüngenden Ende des trichterförmigen Bereiches hin orientiert ist. Mit einer solchen Konstruktion ist eine besonders starke Konzentration der von dem Antriebselement abgegebenen Energie in Richtung der Mittelache der Aspirationsleitung möglich, wobei zusätzlich eine höhere Wellenausbreitungsgeschwindigkeit erreicht wird. Dies führt dazu, dass am distalen Ende der Nadel ein relativ hoher Schalldruck vorliegen kann.

Bei dem erfindungsgemäßen Handstück kann die Nadel an ihrem distalen Ende einen Innendurchmesser von 0,4 bis 1,1 mm besitzen. Bei einem solchen Durchmesser ist noch eine gute Aspiration der erzeugten Linsenpartikel und des Fluides möglich, wobei gleichzeitig nur eine sehr kleine Inzision in die Augenvorderkammer erforderlich ist. Dies bedeutet einen sanfteren operativen Eingriff, geringere Gefahr einer zusätzlichen Sehstörung wie Stabsichtigkeit (Astigmatismus), schnellere Wiederherstellung des Sehvermögens, bessere optische Behandlungsergebnisse und allgemein eine schnellere Heilung und Erholung eines operierten Auges.

Bevorzugt ist das distale Ende der Nadel aus einem Polymerkunstoff gebildet. Da die Nadel bei dem erfindungsgemäßen Handstück nicht mehr schwingen und mit ihrem distalen Ende kein mechanisches Zertrümmern mehr erreichen muss, sind die Anforderungen an eine mechanische Festigkeit des Nadelwerkstoffes deutlich niedriger als bei Handstücken gemäß dem Stand der Technik. So ist es möglich, dass die Nadel aus einem preisgünstigen Polymerkunststoff gebildet ist, welcher zum Beispiel nur für die Einmalverwendung vorgesehen ist. Damit kann ein sehr hohes Sterilitätsniveau und stets ein perfekter Zustand der Nadel erreicht werden. Eine Einmalverwendung ist außerdem vorteilhaft, da sich während einer üblichen Operationsdauer die Spitze der Nadel kaum noch abnutzen kann. Werden Nadeln mehrfach verwendet, wie das bei herkömmlichen Handstücken der Fall ist, rundet sich die Spitze der Nadel mit zunehmender Einsatzdauer immer mehr ab, so dass sie stumpf wird. Dadurch reduziert sich die Effektivität der Phakoemulsifikation, so dass die Nadel bei herkömmlichen Handstücken noch stärker zu Schwingungen angeregt werden muss, um eine akzeptable Emulsifikation der Augenlinse zu erreichen. Durch die Einmalverwendung der Nadel bei dem erfindungsgemäßen Handstück kann ein solcher Nachteil vermieden werden.

Der Polymerkunststoff kann auch ein weicher Polymerkunststoff sein, der sich bei einer Inzision gut an die schlitzförmige Wunde anpassen kann. Damit ist eine noch bessere Abdichtung möglich und eine Schädigung des Gewebes durch starke Zug- oder Druckspannung wird vermieden. Die hohe Abdichtung hat den Vorteil, dass während einer Operation weniger Fluid durch das Auge gepumpt werden muss, so dass sich ein durch eine Operation induziertes Trauma reduzieren lässt.

Weitere Vorteile und Merkmale der Erfindung werden mit Bezug auf die nachfolgenden Zeichnungen erklärt, in welchen zeigen:
- Figur 1: eine schematische Querschnittsdarstellung einer ersten Ausführungsform eines erfindungsgemäßen Handstückes;
- Figur 2: eine schematische Querschnittsdarstellung einer zweiten Ausführungsform des erfindungsgemäßen Handstückes;
- Figur 3: eine schematische Querschnittsdarstellung einer dritten Ausführungsform des erfindungsgemäßen Handstückes; und
- Figur 4: eine schematische Darstellung von Isobaren, die sich beim Einsatz des erfindungsgemäßen Handstückes im Bereich der Nadelspitze ergeben.

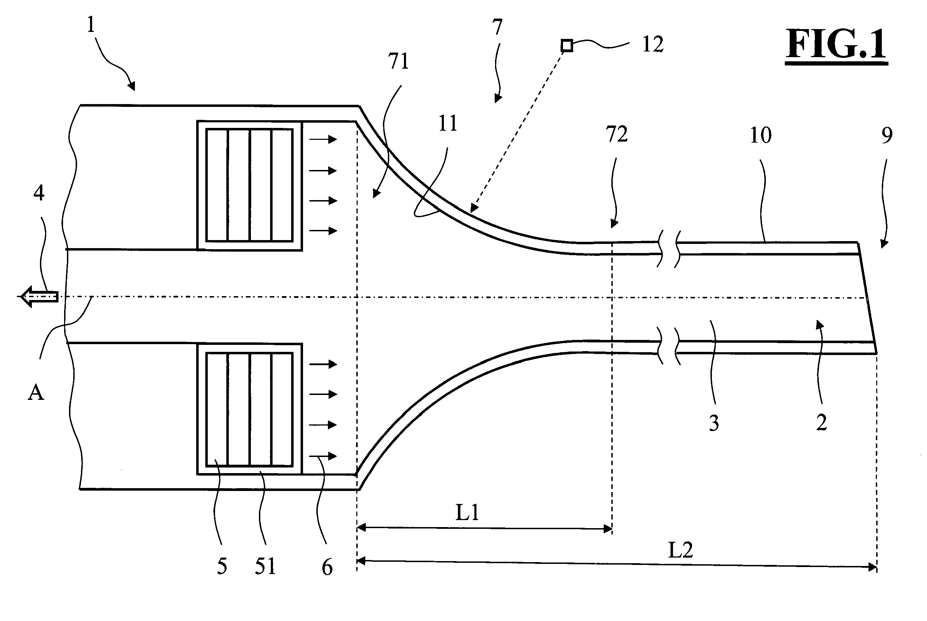

Figur 1 zeigt ein Handstück 1 gemäß einer ersten Ausführungsform der Erfindung. Das Handstück 1 weist eine Nadel 10 mit einer Aspirationsleitung 2 zum Transportieren von Aspirationsfluid 3 auf, welches sich zusammen mit Linsenpartikeln an einem distalen Ende 9 der Nadel 10 ansaugen lässt. Die Absaugung erfolgt dabei vom distalen Ende 9 der Nadel 10 in das Handstück hinein in Richtung des Pfeils 4. Das Handstück 1 weist ferner ein Antriebselement 5 auf, welches innerhalb der Aspirationsleitung angeordnet ist. Bei der in Fig. 1 dargestellten Ausführungsform weist das Antriebselement 5 vier piezoelektrische Elemente auf, welche direkt miteinander verbunden sind. Ein solches Antriebselement 5 ist mit einer elektrischen Isolierung 51 versehen, so dass es zu keinem Kurzschluss mittels des Aspirationsfluides kommen kann.

Das Antriebselement 5 lässt sich so ansteuern, dass eine Longitudinalbewegung in Richtung der Pfeile 6 erzeugt wird. Die von dem Antriebselement 5 abgegebene Bewegungsenergie erzeugt eine Longitudinalwelle, welche direkt in das Aspirationsfluid eingekoppelt wird. Von Bedeutung ist dabei, dass bei dieser Ausführungsform des Handstückes das Antriebselement seine kinetische Energie nicht an die Nadel abgeben kann, sondern nur an das Aspirationsfluid. Somit bleibt die Nadel ohne Bewegung in Ruhe stehen, so dass nur das Aspirationsfluid die Energie zum distalen Ende 9 der Nadel 10 transportieren kann.

Die von dem Antriebselement 5 abgegebene Energie kann besonders gut konzentriert werden, wenn das Aspirationsfluid in einen trichterförmigen Bereich 7 geleitet wird. Bei dieser in Fig. 1 dargestellten ersten Ausführungsform erreicht das Aspirationsfluid ausgehend von dem Antriebselement 5 eine Öffnungszone 71 des trichterförmigen Bereiches 7, woraufhin sich der Querschnitt des trichterförmigen Bereiches 7 zunehmend in Richtung zum distalen Ende 9 der Nadel 10 hin verjüngt, bis schließlich ein Ende 72 des trichterförmigen Bereiches 7 erreicht wird. Bei der in Figur 1 dargestellten Ausführungsform besitzt der trichterförmige Bereich 7 eine Länge L1. Beginnend vom Ende 72 des trichterförmigen Bereiches 7 wird die Nadel 10 in Form eines Hohlzylinders fortgesetzt. Die Gesamtlänge der Nadel 10 einschließlich des trichterförmigen Bereiches 7 besitzt eine Länge L2. Bevorzugt beträgt die Länge L1 = 10 mm und die Länge L2 = 50 mm.

Eine besonders hohe Konzentration der von dem Antriebselement 5 abgegebenen Energie kann erreicht werden, wenn der trichterförmige Bereich 7 im Querschnitt eine Innenwandkontur 11 aufweist, welche zur Mittelachse A der Aspirationsleitung 2 hin so gekrümmt ist, dass ein zugehöriger Krümmungsmittelpunkt 12 zum Ende 72 des trichterförmigen Bereiches 7 hin orientiert ist.

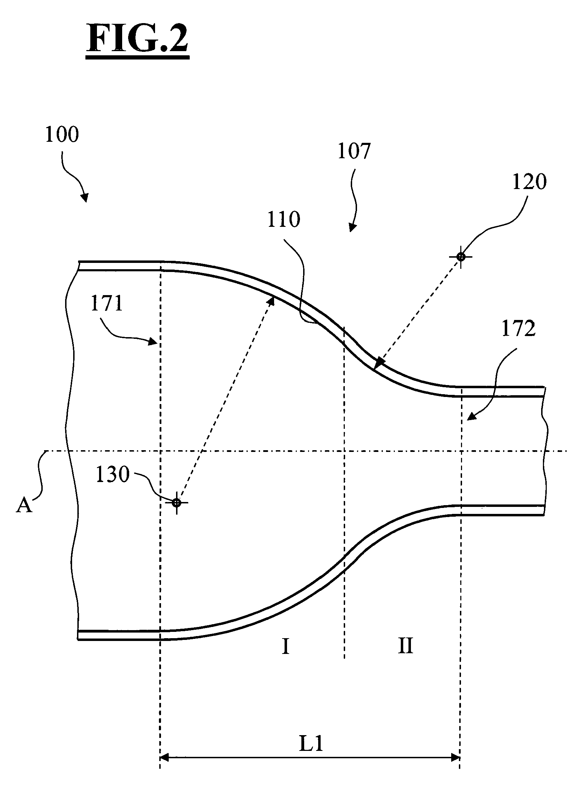

Figur 2 zeigt eine zweite Ausführungsform eines vorderen Teils des erfindungsgemäßen Handstückes. Auch dieses Handstück besitzt einen trichterförmigen Bereich, siehe Bezugszeichen 107, welcher eine erste Zone I und eine zweite Zone II aufweist. Die zweite Zone II ist dabei analog zu dem trichterförmigen Bereich 7 gemäß Figur 1 ausgebildet, indem dieser Bereich 7 eine Innenkontur aufweist, welche zur Mittelachse A der Aspirationsleitung 2 hin so orientiert ist, dass ein zugehöriger Krümmungsmittelpunkt 120 zu einem Ende 172 des trichterförmigen Bereiches 107 hin orientiert ist. Die erste Zone I ist jedoch derart ausgerührt, dass ein ihr zugehöriger Krümmungsmittelpunkt 130 auf einer gegenüberliegenden Seite im Vergleich zur Position des Krümmungsmittelpunktes 120 der zweiten Zone II angeordnet ist. Der Krümmungsmittelpunkt 130 ist somit nicht zum verjüngenden Ende 172 des trichterförmigen Bereiches 107, sondern in Richtung zu einer Öffnungszone 171 des trichterförmigen Bereiches 107 hin orientiert, wobei die Öffnungszone 171 gegenüber dem Ende 172 des trichterförmigen Bereiches 107 angeordnet ist.

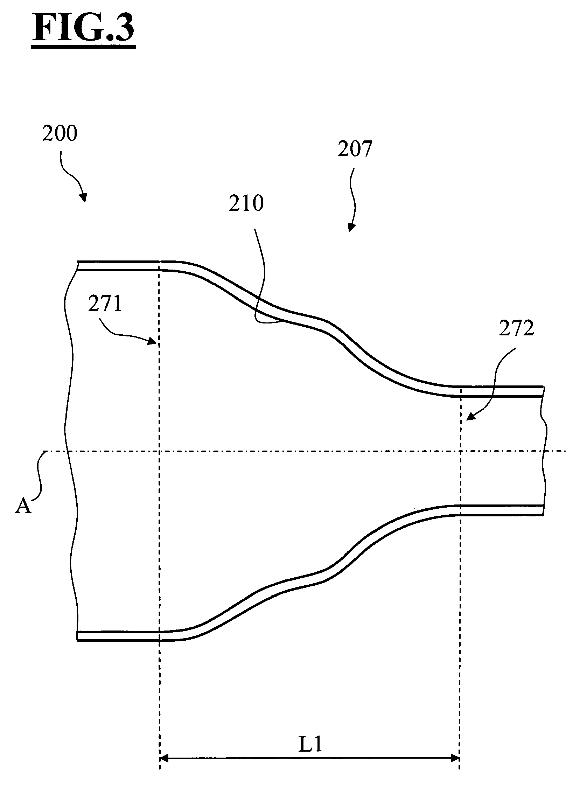

Figur 3 zeigt eine schematische Querschnittsdarstellung einer dritten Ausführungsform eines vorderen Teils des erfindungsgemäßen Handstückes. Ein trichterförmiger Bereich 207 mit einer Öffnungszone 271 und einem Ende 272 ist derart ausgebildet, dass eine zugehörige Innenwandkontur 210 eine Freiformkontur besitzt. Wenn diese Freiformkontur entsprechend ausgebildet ist, treten keine Totwasserbereiche auf, so dass eine hohe Konzentration der von dem Antriebselement 5 abgegebenen Energie in Richtung zum distalen Ende 9 der Nadel 10 erreicht werden kann.

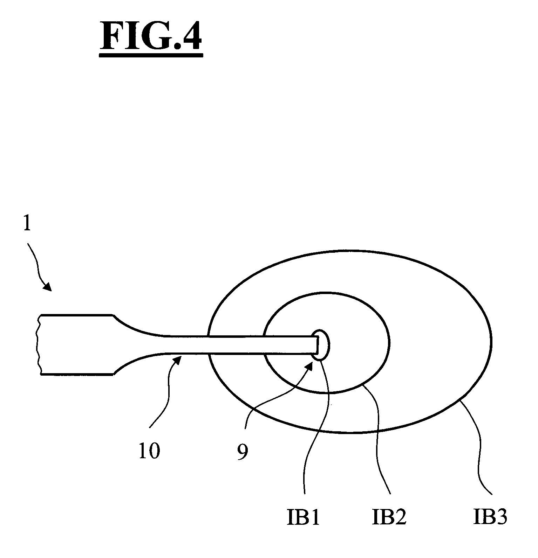

In Figur 4 ist eine Nadel 10 des erfindungsgemäßen Handstückes 1 dargestellt, wobei zusätzlich Isobaren IB1, IB2, IB3 eingezeichnet sind. Bei der Berechnung der Isobaren wurden folgende Randbedingungen berücksichtigt:
Ultraschallwellengeschwindigkeit im Aspirationsfluid: c₀ = 1484 m/s
Ultraschallfrequenz der Antriebselemente: f₀ = 40000 Hz;
Ultraschallwellenlänge im Aspirationsfluid: l₀ = c₀ / f₀ = 37,1 mm;
Amplitude der Antriebselemente: a₀ = 50 µm.

Unter Berücksichtigung dieser Randbedingungen ergibt sich in unmittelbarer Nähe des distalen Endes 9 der Nadel 10 ein Schalldruckpegel von IB1 = 276 dB. Mit diesem Schalldruckpegel wird die zu emulsifizierende Linse direkt beaufschlagt. In einer Entfernung von etwa 7 mm vom distalen Ende 9 der Nadel 10 herrscht ein Schalldruckpegel von etwa IB2 = 200 dB, und in einer Entfernung von etwa 15 mm von der Nadelspitze entfernt herrscht ein Schalldruckpegel von etwa IB3 = 160 dB. Der Schalldruckpegel in unmittelbarer Nähe des distalen Endes 9 der Nadel 10 erreicht bei dem erfindungsgemäßen Handstück einen um etwa 50 dB höheren Betrag, als er bei einem Handstück nach dem Stand der Technik erreichbar ist, wenn die gleichen Randbedingungen zugrunde gelegt werden. Dies bedeutet, dass bei dem erfindungsgemäßen Handstück mit einer unbewegten Nadel ein höherer Schalldruckpegel erzeugt werden kann, um eine Augenlinse zu emulsifizieren, als dies bei einer schwingenden Nadel bei einem herkömmlichen Handstück möglich wäre.

### Bezugszeichenliste:

- 1: Handstück gemäß einer ersten Ausführungsform
- 2: Aspirationsleitung
- 3: Aspirationsfluid
- 4: Absaugrichtung
- 5: Antriebselement
- 51: Isolierung eines Antriebselementes
- 6: Richtung der Longitudinalbewegung
- 7: trichterförmiger Bereich
- 71: Öffnungszone des trichterförmigen Bereiches 7
- 72: verjüngtes Ende des trichterförmigen Bereiches 7
- 9: distales Ende der Nadel 10
- 10: Nadel
- 11: Innenwand des trichterförmigen Bereiches 7
- 12: Krümmungsmittelpunkt der Innenwand 11
- 100: Handstück gemäß einer zweiten Ausführungsform
- 107: trichterförmiger Bereich
- 110: Innenwand des trichterförmigen Bereiches 107
- 120: Krümmungsmittelpunkt der zweiten Zone II des trichterförmigen Bereiches 107
- 130: Krümmungsmittelpunkt der ersten Zone I des trichterförmigen Bereiches 107
- 171: Öffnungszone des trichterförmigen Bereiches 107
- 172: verjüngtes Ende des trichterförmigen Bereiches 107
- 200: Handstück gemäß einer dritten Ausführungsform
- 210: Innenwand
- 207: trichterförmiger Bereich
- 271: Öffnungszone des trichterförmigen Bereiches 207
- 272: verjüngtes Ende des trichterförmigen Bereiches 207
- A: Mittelachse der Aspirationsleitung
- L1: Länge des trichterförmigen Bereiches 7, 107, 207
- L2: Länge der Nadel 10
- IB1, IB2, IB3: Isobare

## Patentansprüche

1. Handstück für die Phakoemulsifikation einer Augenlinse, aufweisend:
- eine Nadel mit einer darin angeordneten Aspirationsleitung zum Transportieren von Aspirationsfluid, und
- mindestens ein Antriebselement zum Erzeugen einer Longitudinalwelle, **dadurch gekennzeichnet, dass** das Antriebselement so angeordnet ist, dass die Longitudinalwelle direkt in das Aspirationsfluid einkoppelbar ist.

2. Handstück nach Anspruch 1, wobei das Antriebselement innerhalb der Aspirationsleitung angeordnet ist und sich in direkten Kontakt mit dem Aspirationsfluid bringbar ist.

3. Handstück nach einem der Ansprüche 1 oder 2, wobei das Antriebselement piezoelektrische Elemente aufweist.

4. Handstück nach einem der vorherigen Ansprüche, wobei das Antriebselement ringförmig ausgebildet ist.

5. Handstück nach einem der vorherigen Ansprüche, wobei die Nadel einen trichterförmigen Bereich mit einem sich verjüngenden Ende aufweist, welches zum distalen Ende der Nadel orientiert ist.

6. Handstück nach Anspruch 5, wobei der trichterförmige Bereich im Querschnitt eine Innenwandkontur aufweist, welche zur Mittelachse der Aspirationsleitung hin so gekrümmt ist, dass ein zugehöriger Krümmungsmittelpunkt zum verjüngenden Ende des trichterförmigen Bereiches hin orientiert ist.

7. Handstück nach einem der vorherigen Ansprüche, wobei die Nadel an ihrem distalen Ende einen Innendurchmesser von 0,4 mm bis 1,1 mm besitzt.

8. Handstück nach einem der vorherigen Ansprüche, wobei das distale Ende der Nadel aus einem Polymerkunststoff gebildet ist.

## Claims

1. Handpiece for the phacoemulsification of an eye lens, comprising:
- a needle with an aspiration line arranged therein for transporting aspiration fluid, and
- at least one drive element for generating a longitudinal wave,
**characterized in that** the drive element is arranged in such a way that the longitudinal wave is directly coupleable into the aspiration fluid.

2. Handpiece according to Claim 1, wherein the drive element is arranged within the aspiration line and directly contactable with the aspiration fluid.

3. Handpiece according to either of Claims 1 and 2, wherein the drive element has piezoelectric elements.

4. Handpiece according to one of the preceding claims, wherein the drive element has a ringshaped embodiment.

5. Handpiece according to one of the preceding claims, wherein the needle has a funnel-shaped region with a tapering end, which is oriented toward the distal end of the needle.

6. Handpiece according to Claim 5, wherein the funnel-shaped region has an internal wall contour in cross section which is curved toward the central axis of the aspiration line in such a way that an associated centre of curvature is oriented toward the tapering end of the funnel-shaped region.

7. Handpiece according to one of the preceding claims, wherein the needle has an internal diameter of 0.4 mm to 1.1 mm at the distal end thereof.

8. Handpiece according to one of the preceding claims, wherein the distal end of the needle is made from polymer plastic.

## Revendications

1. Pièce à main pour la phacoémulsification d'un cristallin oculaire, présentant :
- une aiguille dans laquelle est disposée une ligne d'aspiration pour transporter du fluide d'aspiration, et
- au moins un élément d'entraînement pour générer une ondulation longitudinale,
**caractérisée en ce que**
l'élément d'entraînement est disposé de telle sorte que l'ondulation longitudinale puisse être injectée directement dans le fluide d'aspiration.

2. Pièce à main selon la revendication 1, dans laquelle l'élément d'entraînement est disposé à l'intérieur de la ligne d'aspiration et peut être amené en contact direct avec le fluide d'aspiration.

3. Pièce à main selon l'une quelconque des revendications 1 ou 2, dans laquelle l'élément d'entraînement présente des éléments piézo-électriques.

4. Pièce à main selon l'une quelconque des revendications précédentes, dans laquelle l'élément d'entraînement est réalisé sous forme annulaire.

5. Pièce à main selon l'une quelconque des revendications précédentes, dans laquelle l'aiguille présente une région en forme d'entonnoir avec une extrémité effilée, laquelle est orientée vers l'extrémité distale de l'aiguille.

6. Pièce à main selon la revendication 5, dans laquelle la région en forme d'entonnoir présente, en section transversale un contour de paroi interne, qui est courbé vers l'axe médian de la conduite d'aspiration de telle sorte qu'un centre de courbure associé soit orienté vers l'extrémité effilée de la région en forme d'entonnoir.

7. Pièce à main selon l'une quelconque des revendications précédentes, dans laquelle l'aiguille possède, au niveau de son extrémité distale, un diamètre intérieur de 0,4 mm à 1,1 mm.

8. Pièce à main selon l'une quelconque des revendications précédentes, dans laquelle l'extrémité distale de l'aiguille est formée d'un plastique polymère.
